# EUROPEAN PATENT APPLICATION

(11) **EP 4 470 524 A1**
(43) Date of publication of application: **04.12.2024**
(21) Application number: 23747229.5
(22) Date of filing: 16.01.2023
(51) Int. Cl.: A61K 9/00, A61K 9/16, A61K 47/14, A61K 47/28

(54) **LIPID FUSION DEPOT COMPOSITION WITH CONTROLLED INITIAL RELEASE, AND METHOD FOR PREPARING SAME**

(30) Priority: 27.01.2022 KR 20220012234
(71) Applicant: TIONLAB THERAPEUTICS CO., LTD., Gyeongsangnam-do 50969 (KR)
(72) Inventor: LIM, Duck Soo, Suwon-si Gyeonggi-do 16628 (KR); LEE, Eun Joo, Hwaseong-si Gyeonggi-do 18452 (KR); CHOI, Go Yeong, Suwon-si Gyeonggi-do 16544 (KR); KANG, Chae Lyn, Yongin-si Gyeonggi-do 17094 (KR); JEUN, Su Bin, Seoul 06528 (KR)
(74) Representative: Schiweck Weinzierl Koch Patentanwälte Partnerschaft mbB
(86) International application number: PCT/KR2023/000740
(87) International publication number: WO 2023/146187

(57) **Abstract**

The present invention relates to a depot composition comprising a lipid component inside microspheres. The depot composition is capable of controlling the excessive initial burst of an active ingredient from a formulation containing a biodegradable polymer and an active ingredient and has excellent suspensibility, and thus, even when using same as an injection, a layperson can obtain the effect of the active ingredient uniformly and continuously.

## Description

### [Technical Field]

The present invention relates to a lipid fusion depot composition in which the initial burst is controlled by lipids, and a method for preparing the same.

### [Background Art]

A depot preparation is one of the injectable formulations that prolong the effectiveness of medicine, and is a formulation that is primarily used when administering hormones or the like over a long period of time. Although a depot preparation, which is a sustained-release formulation, has an advantage of minimizing the number of injections required for patients, it is not easy to achieve a constant and sustained release of the drug over the entire release period after injection. In particular, when the initial burst rate is high, in which an excessive amount of drug is released from the depot at the beginning of injection, side effects due to the excess drug may occur and the sustained efficacy of the drug may decrease during the specified period, so it is very important to control the initial burst rate from the depot preparation.

GLP-1 agonists such as liraglutide and semaglutide were first developed to lower blood sugar in diabetic patients, and by confirming that these drugs are also effective in reducing body weight in obese patients, they are used to treat diabetes and obesity. An amount of insulin secreted after a meal is increased by analogs of the incretin hormones secreted in the intestines, and food is allowed to move more slowly from the stomach to the small intestine by increasing the time it takes for the content in the stomach to be released. Further, through various actions of suppressing appetite by acting on the central nervous system, these agonists help to lower blood sugar and reduce body weight.

However, when the body is exposed to an excessive amount of liraglutide, side effects such as nausea, diarrhea, increased heart rate, hypoglycemia or headaches may occur, so it is one of the more important issues to control the initial burst in a depot preparation including liraglutide as an active ingredient.

Meanwhile, Japanese Patent No. 5681626 discloses that a sustained-release formulation including microspheres in which sterol-containing lipid components and polylactide-co-glycolide (PLGA) polymers are combined and a phospholipid component can control the release rate. However, lipids have disadvantages of failing to show a sufficient effect in suppressing the initial burst and causing the properties of the microspheres to deteriorate due to their strong hydrophobic properties. In addition, since the modification of a polylactide-co-glycolide (PLGA) polymer to form microspheres causes difficulties in obtaining approval for drug use, there are limitations in the actual use by applying the corresponding composition to a depot preparation.

Furthermore, in the case of depot preparations, in consideration of the storage safety of the drug, microspheres are stored in a freeze-dried state and suspended in water for injection to be injected. In this case, when the suspension of the microparticles deteriorates, the depot preparation may be administered in an amount less than the prescribed dose of the drug at one time, making it difficult to obtain a sufficient drug effect. Further, poor suspension properties may result in the agglomeration and sedimentation of microspheres, thus the suspension ability of the microspheres is also a very important factor because injection may be difficult due to the phenomenon of needle clogging during injection.

As described above, since sustained-release microspheres have limitations in providing a depot preparation with a sufficiently controlled initial burst, there remains a need for developing a depot composition having suitable resuspensibility and controlled initial burst properties.

### [Disclosure]

### [Technical Problem]

The present invention is directed to providing a depot composition which has excellent resuspensibility and suppresses the excessive initial burst of a drug, and a method for preparing the same.

As a result of making efforts to develop a depot composition capable of controlling the excessive initial burst of a drug, the present inventors confirmed that the initial burst of a drug can be suppressed by containing a lipid component as a release control substance in the oil layer of microspheres. Furthermore, the present inventors confirmed that the microspheres including the lipid component have uniform properties, excellent suspensibility, and thus excellent preparation properties as a depot preparation, thereby completing the present invention.

### [Technical Solution]

One aspect of the present invention provides a depot composition including microspheres including an oil layer (O layer) including a lipid component and a biodegradable polymer.

Another aspect of the present invention provides a method for preparing a depot composition, the method including: preparing an oil phase (O) solution including a lipid component and a biodegradable polymer, or preparing a W1/O emulsion by mixing a first water phase (W1) solution including a water-soluble solvent with the oil phase (O) solution; and
preparing an O/W2 emulsion or a W1/O/W2 emulsion by inputting the oil phase solution or W1/O emulsion into a second water phase 2 (W2) solution.

### [Advantageous Effects]

The microspheres of the depot composition of the present invention can control an active ingredient included in microspheres from being released in an excessive amount at the beginning of the injection of the depot preparation by including a lipid component, and have excellent suspensibility, so that even when the depot composition of the present invention is administered as an injection by a user, the effect of the active ingredient can be obtained uniformly and continuously.

### [Description of Drawings]

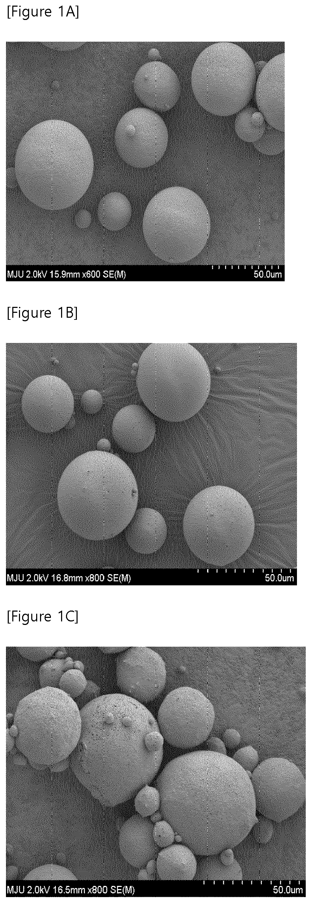

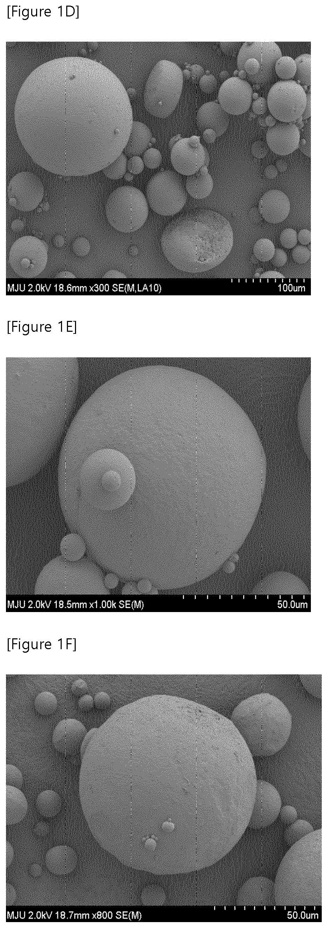

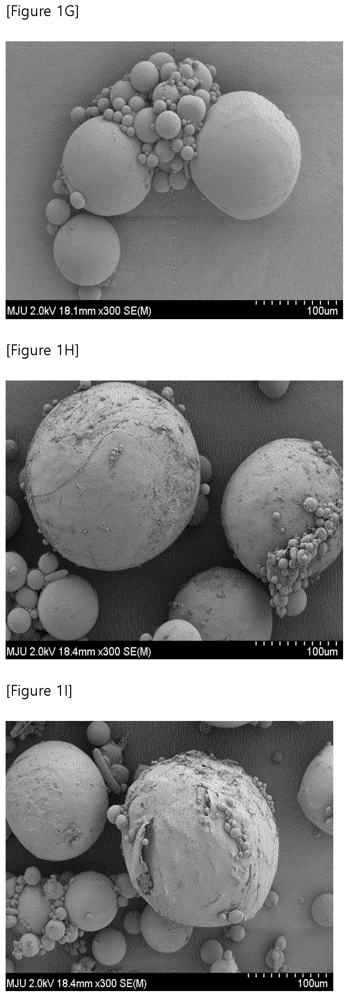

FIGS. 1A to 1I are SEM photographs of microspheres prepared according to an exemplary embodiment of the present invention. Specifically, FIG 1A shows microspheres according to Comparative Example 1-1, FIG 1B shows microspheres according to Comparative Example 1-2, FIG 1C shows microspheres according to Comparative Example 1-3, FIG 1D shows microspheres according to Comparative Example 1-4, FIG 1E shows microspheres according to Example 1-1, FIG 1F shows microspheres according to Example 1-2, FIG 1G shows microspheres according to Example 1-3, FIG 1H shows microspheres according to Example 1-4, and FIG 1I shows microspheres according to Examples 1-5.

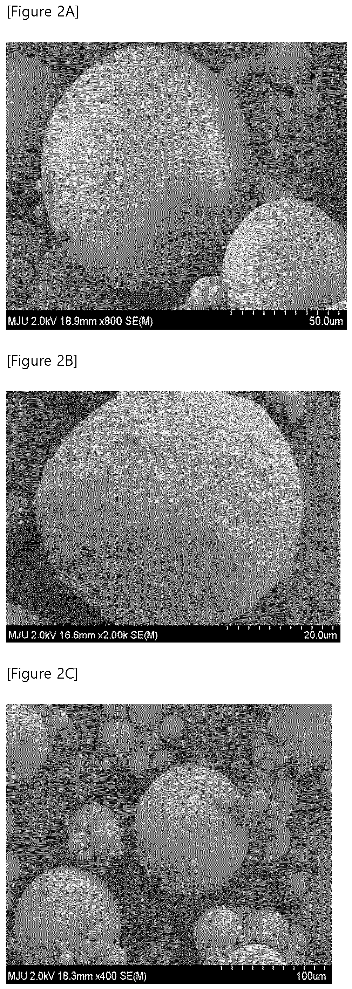

FIGS. 2A to 2D are SEM photographs of microspheres prepared according to an exemplary embodiment of the present invention. Specifically, FIG 2A shows microspheres according to Comparative Example 2-1, FIG 2B shows microspheres according to Comparative Example 2-2, FIG 1C shows microspheres according to Example 2-1, and FIG 1D shows microspheres according to Example 2-2.

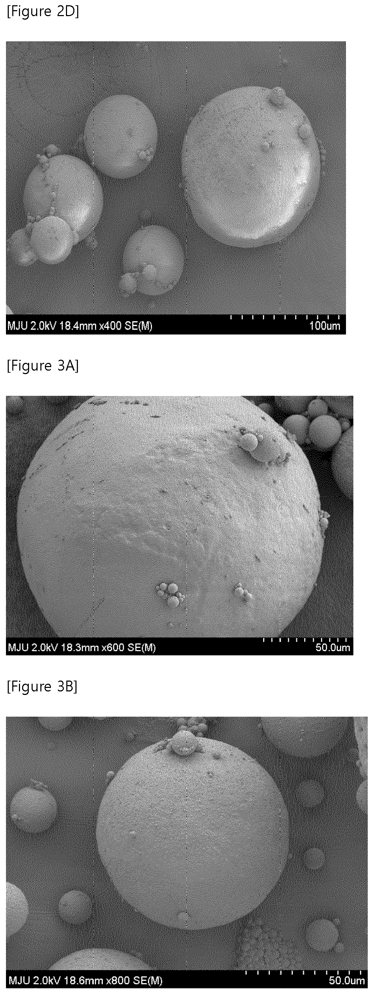

FIGS. 3A and 3B are SEM photographs of microspheres prepared according to an exemplary embodiment of the present invention. Specifically, FIG 3A shows microspheres according to Example 3-1, and FIG 3B shows microspheres according to

### Example 3-2.

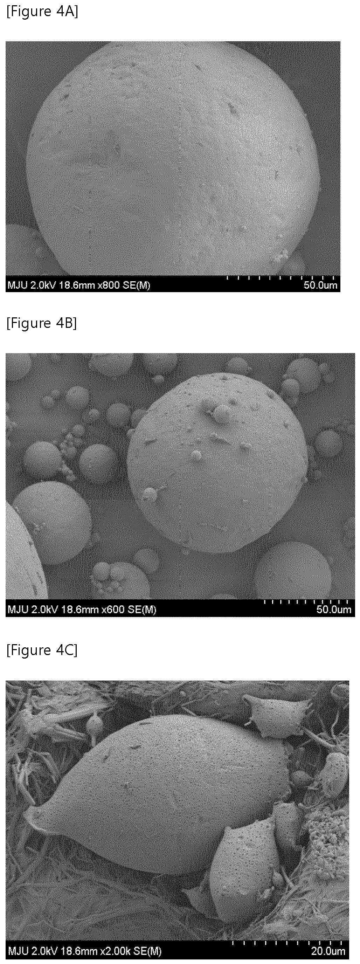

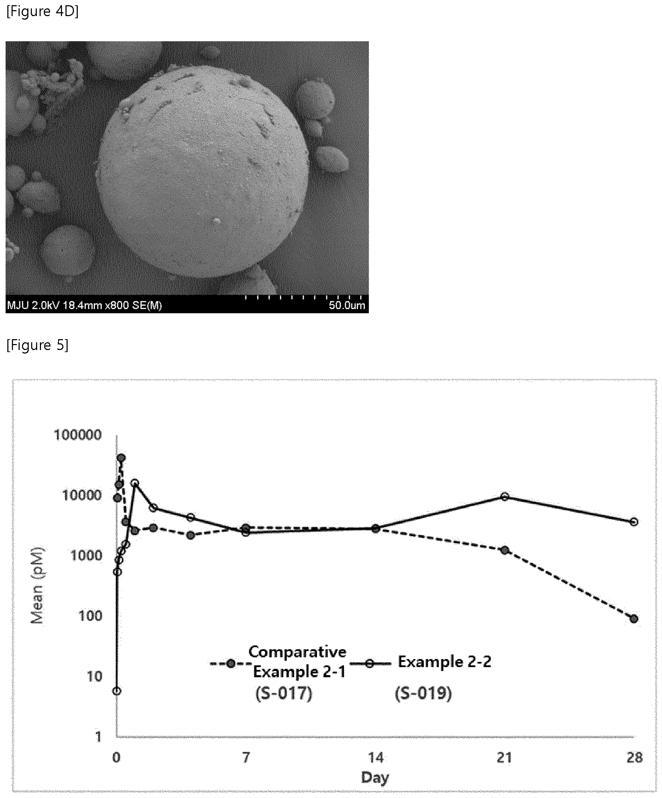

FIGS. 4A to 4D are SEM photographs of microspheres prepared according to an exemplary embodiment of the present invention. Specifically, FIG 4A shows microspheres according to Example 4-1, FIG 4B shows microspheres according to Example 4-2, FIG 4C shows microspheres according to Comparative Example 4-1, and FIG 4D shows microspheres according to Example 4-3.
FIG 5 shows the *in vivo* results of initial burst control and sustained release effects.

### [Modes of the Invention]

Hereinafter, the present invention will be described in detail.

However, since the present invention may be modified into various forms and may have various forms, the specific examples and descriptions set forth below are included merely for aiding the understanding of the present invention and are not intended to limit the present invention to a specific disclosed form. It should be understood that the scope of the present invention includes all the modifications, equivalents, and replacements falling within the spirit and technical scope of the present invention.

In the present invention, the values indicate more or less unless otherwise specified.

The present invention provides a depot composition including microspheres including an oil layer (O layer) including a lipid component and a biodegradable polymer.

The depot composition of the present invention is a formulation capable of releasing a drug for a long period of time, corresponds to a sustained release preparation, and more specifically, may be a sustained release injection preparation. The sustained release formulation needs to gradually release the drug inside at an intended level for a certain period of time. For this purpose, it is necessary to control the initial release (initial burst) in which an excessive amount of the internal drug is released at the beginning of the injection of the depot preparation, and to ensure that the suspension is well formed prior to the injection of the depot preparation. In the present invention, a depot composition was completed by preparing microspheres with excellent suspensibility while initial burst is controlled by mixing lipid components.

The microspheres of the present invention may be in an O/W form, including a single water phase layer (W layer) and a single oil layer (O layer). Further, the microspheres of the present invention may be in a W/O/W form, including a water phase layer (W layer), an oil layer (O layer) and a water phase layer (W layer).

In the present specification, for the convenience of description regarding two or more water phase layers or water phase solutions, a water phase layer present in the oil layer of the microsphere or a water phase solution forming the water phase layer may be referred to as a first water phase layer (W1 layer) or a first water phase solution, and a water phase layer formed on the outer periphery of the oil phase layer of the microsphere or a water solution forming the water phase layer may be referred to as a second water phase layer (W2 layer) or a second water phase solution. In this case, the water phase layer (W layer) or water phase solution in the microspheres in an O/W form is understood to have a structure corresponding to a second water phase layer (W2 layer) or second water phase solution in the microspheres in an W1/O/W2 form. Therefore, the O/W emulsion may also be referred to as an O/W2 emulsion in the present specification, and in the following description, reference to the second water phase layer (W2 layer) or the second water phase solution should be understood as a description corresponding to the water phase layer or water phase solution for the microspheres in an O/W form.

The microspheres may be prepared by a single emulsification method of producing an O/W emulsion by adding an oil phase solution including a lipid component and a biodegradable polymer to a water phase solution and emulsifying the resulting mixture. In addition, the microspheres may be prepared by a double emulsification method of producing a W/O/W emulsion by adding a W/O emulsion to a water phase solution and emulsifying the resulting mixture.

Specifically, the microspheres of the present invention may be prepared by a method of preparing an oil phase (O) solution by dissolving a lipid component and a biodegradable polymer in a fat-soluble solvent; or preparing a W1/O emulsion by mixing a first water phase 1 (W1) solution prepared by dissolving an active ingredient in a water-soluble solvent with an oil phase (O) solution prepared by dissolving the biodegradable polymer and the lipid component in a fat-soluble solvent; and
preparing an emulsion by inputting the oil phase solution or W1/O emulsion into a second water phase 2 (W2) solution including a water phase solvent.

As the fat-soluble solvent of the present invention, any pharmaceutically acceptable carrier or solvent typically used in the technical field of the present invention may be used. As an example, dichloromethane may be used, but the solvent is not limited thereto.

In the present invention, as the water-soluble solvent, any pharmaceutically acceptable carrier or solvent typically used in the technical field of the present invention may be used. As an example, polyvinyl alcohol (PVA) or sodium acetate may be used, but the solvent is not limited thereto. The second aqueous solution may further include an osmotic pressure adjusting agent such as sodium chloride to adjust the osmotic pressure of the solution during the emulsion preparation process.

In the present invention, when a lipid component is included in the oil layer of the microspheres, the depot composition may be effectively used as an injection by significantly improving the suspensibility of the depot composition along with the initial burst suppression effect of suppressing the excessive release of the active ingredient in the microspheres.

The microspheres of the present invention may have a bulk density (BD) value of 0.1 g/ml or more.

The bulk density (BD) refers to a density based on a volume including voids generated between particles when a specific container is filled with a powder or granular material. When the bulk density of the microspheres in the depot composition is less than 0.1 g/ml, the microspheres are so light that it is difficult to use the depot composition as an injection. In particular, due to the bulky nature of the microspheres, there is a problem of poor dispersion in water for injection when resuspended. The microspheres of the present invention have a bulky density of 0.1 g/ml or more, and have excellent resuspensibility and excellent properties as an injection.

The microspheres of the present invention may have a zeta potential value of 8 mV or more as an absolute value.

The zeta potential is a unit expressing the magnitude of the repulsive and attractive forces between particles, and the microspheres of the present invention may have a zeta potential value of 8 mV or more as an absolute value. When the zeta potential value is less than 8 mV, the microspheres have reduced dispersibility, and thus, may settle in the water for injection.

Preferably, the microspheres of the present invention have a zeta potential value of 8 mV to 40 mV or 10 mV to 35 mV as an absolute value. When the above range is satisfied, the microspheres have excellent dispersibility, and thus, can be well suspended in water for injection, and in this case, a uniform depot preparation may be formed, so it is possible to provide excellent properties, particularly, as an injection preparation.

The microspheres of the present invention further include an active ingredient. That is, since the depot composition of the present invention is administered into the body, and serves to release the active ingredient into the body for a certain period of time, the active ingredient may be included in the depot composition. In particular, the active ingredient is included in the microspheres to be protected from the microspheres, and may remain in the body for a certain period of time.

The active ingredient may be included in the first water phase layer (W1 layer) or in the oil layer (O layer) of the microspheres of the present invention. Typically, in the case of a single oil emulsion, the active ingredient may be included in the oil layer, and in the case of a double oil emulsion, the active ingredient may be included in the first water phase layer.

The active ingredient used for the purpose of release in the present invention may be a drug for treating a specific disease, symptom or condition, and more preferably, a biopharmaceutical or peptide drug. The drug may be, as a specific example, liraglutide or semaglutide.

The liraglutide and semaglutide may be used for the treatment of obesity or diabetes as they are GLP-1 agonists involved in the action of glucagon-like peptide-1 (GLP-1), which is a hormone that serves to lower blood sugar. When the body is exposed to an excessive amount of liraglutide or semaglutide, symptoms such as vomiting, nausea, hypoglycemia and headaches may occur.

Therefore, when the depot composition includes liraglutide or semaglutide as an active ingredient, it is very important to prevent the active ingredient from being released in an excessive amount from the depot preparation. Furthermore, liraglutide or semaglutide is a peptide drug and is difficult to store and preserve, and to address this, the depot composition may be dried and stored in a powder form. In this case, the powder needs to be suspended in water for injection for the purpose of injection, but when the powder has poor suspensibility, a uniform preparation cannot be formed, resulting in a decrease in the uniformity of drug efficacy, and agglomeration and sedimentation of the microspheres may occur or floating microspheres may occur, making it difficult to secure the initial burst control effect. Therefore, when liraglutide or semaglutide is included as an active ingredient, it is very important to implement a preparation that suppresses initial burst and has excellent suspensibility.

In order to provide a depot composition having such an ability to control initial burst, the oil layer (O layer) of the microspheres of the present invention includes a lipid component and a biodegradable polymer.

The lipid component may be GDO or tripalmitin. Further, the lipid component may be a mixture of cholesterol and a second lipid component. Such a second lipid component may be one or more selected from the group consisting of GDO, DOTAP and tripalmitin. When the oil layer of the microspheres of the present invention includes a lipid component, the pores on the surface of the microspheres are reduced by the lipid component, and due to water-incompatible nature of the lipid component in the oil layer, the active ingredient present in the first water phase layer or oil layer may be partially suppressed from coming out of the oil layer, thereby controlling initial burst.

In addition, when the oil layer includes the lipid component, there is an advantage in that even when the lipid component is included at a low content, an effective initial burst control effect can be obtained, and the suspensibility of the depot can be significantly improved.

The microspheres may be prepared by including 20 parts by weight or less of the lipid component with respect to 100 parts by weight of the biodegradable polymer in the oil phase solution. When the lipid component is mixed in an amount exceeding 20 parts by weight, there is a problem in that the uniformity of the depot drug decreases due to the non-uniformity of the surface of the microspheres.

More specifically, the microspheres may be prepared by including 0.1 to 20 parts by weight, 0.1 to 10 parts by weight, 0.5 to 5 parts by weight, 0.5 to 4.5 parts by weight, or 1 to 4.5 parts by weight of the lipid component with respect to 100 parts by weight of the biodegradable polymer in the oil phase solution. In this case, the microspheres have an excellent effect of suppressing the initial burst of the active ingredient.

Furthermore, cholesterol may be included in an amount of more than 0.001 moles and less than 50 moles, 0.002 moles to 40 moles, 0.002 moles to 20 moles, or 0.1 moles to 1 mole with respect to 1 mole of the second lipid component.

The biodegradable polymer has the characteristics of being dgraded *in vivo,* and has the characteristics of forming microspheres in the present invention, and as a polymer is gradually degraded *in vivo,* the drug included therein may be gradually released. That is, the biodegradable polymer serves to protect the drug therein during the drug release period and control the drug release over a long period of time.

The biodegradable polymers specifically approved for use in a preparation for injection by the Ministry of Food and Drug Safety of each country may be used without limitation in the present invention.

The biodegradable polymer may have an inherent viscosity of 0.35 dL/g to 0.65 dL/g, preferably 0.50 dL/g to 0.55 dL/g. When the inherent viscosity is less than 0.35 dL/g, the drug may be released faster than the desired period, whereas when the inherent viscosity is more than 0.65 dL/g, the drug may be released slower than the desired period, making it difficult to obtain a sufficient drug effect. As a preferred example, a polymer having a viscosity of 0.53 dL/g can be used to maintain drug release characteristics for a long period of time as a depot composition, thereby enabling bioavailability. The inherent viscosity may be measured by a viscosity measuring method for polylactide-co-glycolide (PLGA) provided by the manufacturer.

Specifically, the biodegradable polymer may be a polymer including lactide and glycolide as monomers. Any polymer is included in the present invention regardless of the polymerized form as long as it includes lactide and glycolide as monomers. Further, the polymer also includes a branched polymer in which the ends of the polymer are modified. An example of the polymer may be selected from the group consisting of polylactide (PLA), polyglycolide (PGA), polylactide-co-glycolide (PLGA), and glucose-PLGA, but is not limited thereto. In this regard, the microspheres of the invention may be referred to as PLGA microspheres.

The biodegradable polymer may have the following molecular weight distribution content for each of these molecular weights determined by gel permeation chromatography analysis: the molecular weight range of 500 to 4,000 is 3% or more, the molecular weight range of 5,000 to 16,000 is 10% or more and less than 30%, the molecular weight range of 16,000 to 40,000 is 20% or more and less than 50%, and the molecular weight range of 40,000 or more is 20% or more.

The depot composition of the present invention may additionally include a pharmaceutically acceptable carrier or excipient, and the like. However, preferably, the depot composition of the present invention may not include a stabilizer, a pH adjusting agent, and oxidizing agent.

In addition, the present invention provides a method for preparing a depot composition, the method including: preparing an oil phase (O) solution including a lipid component and a biodegradable polymer, or preparing a W1/O emulsion by mixing a first water phase (W1) solution including a water-soluble solvent with the oil phase (O) solution; and preparing an O/W2 emulsion or a W1/O/W2 emulsion by inputting the oil phase solution or W1/O emulsion into a second water phase 2 (W2) solution.

The present invention may further include recovering microspheres by drying and/or filtering the prepared emulsion and then centrifuging the emulsion.

The first water phase 1 (W1) solution may be prepared by dissolving an active ingredient in a water-soluble solvent. In the present invention, as the water-soluble solvent, any pharmaceutically acceptable carrier or solvent typically used in the technical field of the present invention may be used. As an example, polyvinyl alcohol (PVA) or sodium acetate may be used, but the solvent is not limited thereto.

The active ingredient refers to a drug included in the depot composition of the present invention for the purpose of being released, and may be used in the present invention without being limited to its type. Specifically, the active ingredient may be a water-soluble drug, a biopharmaceutical or a peptide drug. As an example, the active ingredient may be liraglutide or semaglutide.

The oil phase (O) solution may be prepared by mixing and dissolving a lipid component and a biodegradable polymer in a fat-soluble solvent. Furthermore, the active ingredient may also be mixed and dissolved in the oil phase solution. In particular, in the case of microspheres prepared by a single emulsification method, an emulsion may be prepared while adding an oil phase solution in which the active ingredient is mixed to a water phase solution.

As the solvent for including the active ingredient in the oil phase solution, a solvent typically used in the technical field of the present invention may be used.

As the fat-soluble solvent of the present invention, any pharmaceutically acceptable carrier or solvent typically used in the technical field of the present invention may be used. As an example, dichloromethane may be used, but the solvent is not limited thereto.

The lipid component of the present invention may be GDO or tripalmitin. Further, the lipid component may be a mixture of cholesterol and a second lipid component. Such a second lipid component may be one or more selected from the group consisting of GDO, DOTAP and tripalmitin.

The lipid component may be included in the oil solution in an amount of 20 parts by weight or less, 0.1 to 20 parts by weight, 0.1 to 10 parts by weight, 0.5 to 5 parts by weight, 0.5 to 4.5 parts by weight, or 1 to 4.5 parts by weight with respect to 100 parts by weight of the biodegradable polymer. In this case, the prepared microspheres have an excellent effect of suppressing the initial burst of the active ingredient. When the lipid component is included in the oil phase solution in an amount of more than 5 parts by weight, the surface of the microspheres becomes non-uniform, so there is a problem in that the uniformity of the depot drug deteriorates.

In addition, the lipid component may be included in the oil phase solution in an amount of 0.01 to 10 moles with respect to 1 mole of the active ingredient.

Furthermore, cholesterol may be included in an amount of more than 0.001 moles and less than 50 moles, 0.002 moles to 40 moles, 0.002 moles to 20 moles, or 0.1 moles to 1 mole with respect to 1 mole of the second lipid component.

The biodegradable polymer of the present invention may be a polymer including lactide and glycolide as monomers. The polymer including lactide and glycolide as monomers is as described above.

In the present invention, the O/W emulsion may be prepared by mixing an oil phase in a water phase and stirring the resulting mixture with a homogenizer. The stirring speed and time may differ depending on the emulsion forming conditions and the amount of sample.

The second water phase 2 (W2) solution may be prepared by including a water-soluble solvent.

In the present invention, as the water-soluble solvent, any pharmaceutically acceptable carrier or solvent typically used in the technical field of the present invention may be used. As an example, polyvinyl alcohol (PVA) or sodium acetate may be used, but the solvent is not limited thereto. The second aqueous solution may further include an osmotic pressure adjusting agent such as sodium chloride to adjust the osmotic pressure of the solution during the emulsion preparation process.

Further, the water-soluble solvent may further include polyvinyl alcohol, methylcellulose, polyvinylpyrrolidone, carboxymethylcellulose, lecithin, gelatin, polyoxyethylene, polyoxyethylene sorbitan fatty acid esters, polyoxyethylene castor oil derivatives, and mixtures thereof.

In addition, the present invention provides a depot composition including microspheres prepared by the aforementioned preparation method. In this case, it is possible to obtain a depot composition having an excellent effect of suppressing the initial burst of the active ingredient in the microspheres and excellent suspensibility.

Hereinafter, the present invention will be described in detail through examples. However, the following examples are only for exemplifying the present invention, and the scope of the present invention is not limited to the following examples. These examples are provided to make the disclosure of the present invention complete and to allow a person skilled in the art to which the present invention belongs to completely comprehend the scope of the present invention, and the present invention is defined only by the scope of the claims.

### Examples

### Preparation Example 1. Preparation of microspheres

A first water phase (W1) solution was prepared by dissolving 252 mg of liraglutide (Polypeptide Laboratories) as an API in 1.2 mL of a 1 wt% sodium acetate (Daejung Chemicals & Metals Co. Ltd.) solution. Further, a first water phase 1 (W1) solution was prepared by dissolving 252 mg of semaglutide as an API in 1.2 mL of primary and tertiary distilled water.

An oil phase (O) solution was prepared by dissolving 1,350 mg of a DL-lactic acid-glycolic acid copolymer (poly D,L-lactide-co-glycolide; Resomer select 5545 DLG 5Glu, Evonik; inherent viscosity 0.53 dl/g) and a lipid component in 5 mL of dichloromethane (Honeywell). A W1/O emulsion was prepared by mixing the W1 solution and the O solution and stirring the resulting mixture with a homogenizer at 9000 rpm for 2 minutes.

Next, a second water phase 2 (W2) solution was prepared by dissolving 0.5 g of sodium chloride (NaCl; Daejung Chemicals & Metals Co. Ltd) in 100 mL of a 0.5 wt% polyvinyl alcohol (PVA; Gohsenol EG-40P, Nippon Gohsei) solution. A W1/O/W2 emulsion was prepared by injecting the W1/O emulsion into the W2 solution at a rate of 5 ml/min while stirring at 9000 rpm with a homogenizer. The prepared W1/O/W2 emulsion was dried in water at room temperature for 3 hours, filtered through a sieve with a mesh size of 75 µm, and then centrifuged to recover microspheres. After the recovered microspheres were redispersed in distilled water, the surfaces of the microspheres were washed three times by centrifugation. The washed microspheres were freeze-dried to finally obtain drug-loaded microspheres.

### [Test Methods]

### 1. Evaluation of liraglutide or semaglutide content

Liraglutide was quantified by putting 20 mg of microspheres into a 20 mL volumetric flask, dissolving the microspheres in 10 mL of acetonitrile, adjusting the marked line with a 1 wt% sodium acetate solution, then filtering through a 0.45 syringe filter, and then performing high-performance liquid chromatography. In this case, measurement was performed under analytical conditions of 215 nm and a flow rate of 1.0 mL/min using an Aegispak C18-L column employing a solvent in which 0.05M potassium phosphate monobasic and acetonitrile were mixed at a ratio of 53:47 as the mobile phase.

Meanwhile, semaglutide was quantified by putting 20 mg of microspheres into a 20 mL volumetric flask, dissolving the microspheres in 10 mL of acetonitrile, adjusting the marked line with tertiary distilled water, then filtering through a 0.45 syringe filter, and then performing high-performance liquid chromatography. In this case, measurement was performed under analytical conditions of 200 nm and a flow rate of 0.8 mL/min using an Aegispak C18-L column employing a solvent in which mobile phase A of 0.1% TFA:acetonitrile = 90:10 and mobile phase B of 0.1% TFA:acetonitrile = 10:90 were mixed at a ratio of 55:45 as the mobile phase.

### 2. Release test

A release test was carried out using 20 mg of freeze-dried microspheres in 100 mL of a phosphate buffer (1XPBS) containing 0.05% Polysorbate 80 at 120 rpm and 37°C in a DISTEK eluter (Dissolution System 2500). A 2-mL sample was collected and then centrifuged to separate the supernatant from the microspheres, and the amount of liraglutide or semaglutide present in the supernatant was quantified by high-performance liquid chromatography.

In this case, measurement was performed under liraglutide analysis conditions of 215 nm and a flow rate of 1.0 mL/min using an Aegispak C18-L column employing a solvent in which 0.05M potassium phosphate and acetonitrile were mixed at a ratio of 53:47 as the mobile phase. Further, measurement was performed under semaglutide analysis conditions of 200 nm and a flow rate of 0.8 mL/min using an Aegispak C18-L column employing a solvent in which mobile phase A of 0.1% TFA:acetonitrile = 90:10 and mobile phase B of 0.1% TFA:acetonitrile = 10:90 were mixed at a ratio of 55:45 as the mobile phase.

For the initial burst of drug from the microspheres, the amount of liraglutide or semaglutide eluted for 1 hour was confirmed using the aforementioned method, and for long-term release, the amount of liraglutide or semaglutide eluted for 35 days was determined.

### 3. SEM measurements

After about 10 mg of the microspheres were fixed on an aluminum stub and coated with platinum under a vacuum of 0.1 torr and high voltage (10 kV) for 3 minutes, the surface of the microspheres was observed by mounting the microspheres on a SEM powder or granular material and using an image analysis program.

### 4. Particle size measurements

The size of microspheres was measured using a Mastersizer manufactured by Malvern Panalytical Ltd. About 30 mg of microspheres were suspended in 5 ml of distilled water, the suspension was put into a dispersing device and dispersed under the conditions of 2800 rpm and 50% ultrasound for 1 minute, and then the size was measured.

### 5. Zeta-potential measurements

After 50 mg of microspheres were dispersed in 3 ml of purified water, the zeta potential was measured using a Nano-ZS device manufactured by Malvern Panalytical Ltd.

### 6. Bulk density measurements

After an Eppendorf tube was filled with 1 ml of microspheres, the mass of the filled microspheres was measured.

### [Test Example 1] Evaluation of effect of mixing liraglutide as main component with lipid-based excipients

The present inventors conducted an experiment for confirming whether the excess release of the drug could be controlled depending on the type of lipid in microspheres including liraglutide as the main component. Microspheres were prepared with the ingredients and contents shown in the following Table 1. The method for preparing the microspheres is the same as that for preparing the microspheres in Preparation Example 1.

**[Table 1]**

| | **Batch No.** | **Composition** | | | |
|---|---|---|---|---|---|
| | | **W1 Phase (mg)** | **O Phase (mg)** | | **W2 Phase (g)** |
| | | API | Polymer | Lipid | PVA |
| Comparati ve Example 1-1 | 111 | 252 | 1350 | - | 0.5 |
| Comparati ve Example 1-2 | 113 | 252 | 1350 | Stearic acid 19 mg | 0.5 |
| Comparati ve Example 1-3 | 115 | 252 | 1350 | Cholesterol 26 mg | 0.5 |
| Comparati ve Example 1-4 | 117 | 252 | 1350 | DOTAP 47 mg | 0.5 |
| Example 1-1 | 118 | 252 | 1350 | 1,3-GDO 42 mg | 0.5 |
| Example 1-2 | 119 | 252 | 1350 | Tripalmitin 54 mg | 0.5 |
| Example 1-3 | 124 | 252 | 1350 | Cholesterol 26 mg + DOTAP 47 mg | 0.5 |
| Example 1-4 | 125 | 252 | 1350 | Cholesterol 26 mg + 1,3-GDO 42 mg | 0.5 |
| Example 1-5 | 126 | 252 | 1350 | Cholesterol 26 mg + Tripalmitin 54 mg | 0.5 |

To confirm the effect of suppressing the initial burst in microspheres including no lipid component (Comparative Example 1-1), microspheres including stearic acid (Comparative Example 1-2), microspheres including cholesterol (Comparative Example 1-3), microspheres including DOTAP (Comparative Example 1-4), microspheres including 1,3-GDO (Example 1-1), microspheres including tripalmitin (Example 1-2), microspheres including cholesterol and DOTAP (Examples 1-3), microspheres including cholesterol and 1,3-GDO (Example 1-4), and microspheres including cholesterol and tripalmitin (Example 1-5) according to Table 1, the content and release rate of liraglutide (API) were confirmed under the conditions of Test Methods 1 and 2, the surface of the microspheres was observed using SEM photographs under the conditions of Test Methods 3 and 4, and the average particle size (D[4,3]) of each of the microspheres was measured.

The results are shown in the following Table 2 and FIG 1.

FIGS. 1A to 1I are SEM photographs of microspheres prepared according to an exemplary embodiment of the present invention. Specifically, FIG 1A shows microspheres according to Comparative Example 1-1, FIG 1B shows microspheres according to Comparative Example 1-2, FIG 1C shows microspheres according to Comparative Example 1-3, FIG 1D shows microspheres according to Comparative Example 1-4, FIG 1E shows microspheres according to Example 1-1, FIG 1F shows microspheres according to Example 1-2, FIG 1G shows microspheres according to Example 1-3, FIG 1H shows microspheres according to Example 1-4, and FIG 1I shows microspheres according to Examples 1-5.

**[Table 2]**

| | **Batch No.** | **Analysis results** | | | |
|---|---|---|---|---|---|
| | | **Content (%)** | **Release (1h) (%)** | **D[4,3] (µm)** | **SEM properties** |
| Comparative Example 1-1 | 111 | 100.51 | 15.76 | 42.29 | Many pores |
| Comparative Example 1-2 | 113 | 104.85 | 10.70 | 37.75 | Pores present |
| Comparative Example 1-3 | 115 | 106.94 | 18.66 | 39.86 | Many pores |
| Comparative Example 1-4 | 117 | 81.84 | 18.06 | 88.09 | Pores present |
| Example 1-1 | 118 | 101.43 | 9.41 | 60.48 | No pores (coating) |
| Example 1-2 | 119 | 93.99 | 9.75 | 50.82 | No pores (coating) |
| Example 1-3 | 124 | 89.27 | 6.92 | 71.59 | No pores (coating) |
| Example 1-4 | 125 | 104.31 | 5.30 | 73.12 | No pores (coating) |
| Example 1-5 | 126 | 86.74 | 5.65 | 73.05 | No pores (coating) |

As shown in Table 2 and FIGS. 1A to 1I, it can be confirmed that in the microspheres of Comparative Examples 1-1 to 1-4, which do not include a lipid component, or the microspheres including stearic acid, cholesterol, or DOTAP as a lipid component, many pores are present on the surface of the microspheres, and accordingly, a large amount of API is also initially released.

However, it can be seen that when GDO or tripalmitin is used as the lipid component in Examples 1-1 to 1-5, or a mixture of cholesterol and GDO, tripalmitin or DOTAP is used, the pores of the microspheres are covered, and thus, almost no pores are confirmed on the surface. Through this, it can be confirmed that properties are also good while having the effect of suppressing the initial burst of API.

In particular, it can be confirmed that the microspheres in which cholesterol and the second lipid component are combined in Examples 1-3 to 1-5 have an excellent effect of suppressing the initial burst of API.

### [Test Example 2] Evaluation of effect of mixing semaglutide as main component with lipid-based excipients

The present inventors conducted an experiment for confirming whether the excess release of the drug could be controlled depending on the type of lipid in microspheres including semaglutide as the main component. Microspheres were prepared with the ingredients and contents shown in the following Table 3. The method for preparing the microspheres is the same as that for preparing the microspheres in Preparation Example 1.

**[Table 3]**

| | **Batch No.** | **Composition** | | | |
|---|---|---|---|---|---|
| | | **W1 Phase (mg)** | **O Phase (mg)** | | **W2 Phase (g)** |
| | | API | Polymer | Lipid | PVA |
| Comparative Example 2-1 | S-017 | 252 | 1350 | - | 0.5 |
| Comparative Example 2-2 | S-Chol | 252 | 1350 | Cholesterol 26 mg | 0.5 |
| Example 2-1 | S-018 | 252 | 1350 | 1,3-GDO 42 mg | 0.5 |
| Example 2-2 | S-019 | 252 | 1350 | 1,3-GDO 26 mg+ Cholesterol 42 mg | 0.5 |

To confirm the effect of suppressing the initial burst in microspheres including no lipid component (Comparative Example 2-1), microspheres including cholesterol (Comparative Example 2-2), microspheres including 1,3-GDO (Example 2-1), and microspheres including cholesterol and 1,3-GDO (Example 2-2) according to Table 3, the content and release rate of semaglutide (API) were confirmed under the conditions of Test Methods 1 and 2, the surface of the microspheres was observed using SEM photographs under the conditions of Test Methods 3 and 4, and the average particle size (D[4,3]) of each of the microspheres was measured.

In addition, the physicochemical properties (bulk density (BD), zeta potential) of each of the microspheres were measured using Test Methods 5 and 6.

The results are shown in the following Table 4 and FIG 2.

FIGS. 2A to 2D are SEM photographs of microspheres prepared according to an exemplary embodiment of the present invention. Specifically, FIG 2A shows microspheres according to Comparative Example 2-1, FIG 2B shows microspheres according to Comparative Example 2-2, FIG 2C shows microspheres according to Example 2-1, and FIG 2D shows microspheres according to Example 2-2.

**[Table 4]**

| | **Batch No.** | **Analysis results** | | | | | |
|---|---|---|---|---|---|---|---|
| | | **Content (%)** | **Release (1h) (%)** | **D[4,3] (µm)** | **SEM properties** | **Zeta value (mV)** | **BD (g/ml)** |
| Compara tive Example 2-1 | S-017 | 108.66 | 18.11 | 49.28 | Pores present | -7.86 | 0.251 |
| Compara tive Example 2-2 | S-Chol | 101.64 | 21.66 | 39.28 | Many pores | -10.86 | 0.241 |
| Example 2-1 | S-018 | 107.93 | 6.91 | 45.11 | No pores (coating) | -11.7 | 0.229 |
| Example 2-2 | S-019 | 109.42 | 6.56 | 56.68 | No pores (coating) | -13.8 | 0.279 |

As shown in Table 4 and FIGS. 2A to 2D, it can be confirmed that there are many pores on the surface of the microspheres including no lipid component in Comparative Example 2-1 and the microspheres including cholesterol alone in Comparative Example 2-2. Furthermore, it can be confirmed that the microspheres of Comparative Example 2-2 also had poor properties, and accordingly, the initial burst of API was also high.

However, it can be seen that when GDO was used alone as the lipid component in Example 1-1 or a mixture of cholesterol and GDO was used in Example 1-2, almost no pores were confirmed on the surface because the pores of the microspheres were covered. Through this, it can be confirmed that properties are also good while having the effect of suppressing the initial burst of API.

### [Test Example 3] Confirmation of effect of suppressing release control depending on content of 1,3-GDO

In order to confirm whether the effect of suppressing the release of the drug in the microspheres including semaglutide as the main component from the microspheres is caused by the content of the lipid component, the release of API from the microspheres and the properties of the microspheres depending on the content of 1,3-GDO were confirmed.

Microspheres were prepared with the ingredients and contents shown in the following Table 5. The method for preparing the microspheres is the same as that for preparing the microspheres in Preparation Example 1.

**[Table 5]**

| | **Batch No.** | **Composition** | | | |
|---|---|---|---|---|---|
| | | **W1 Phase (mg)** | **O Phase (mg)** | | **W2 Phase (g)** |
| | | API | Polymer | Lipid | PVA |
| Example 3-1 | S-020 | 252 | 1350 | 1,3-GDO 4.2 mg (0.1 moles) | 0.5 |
| Example 2-1 | S-018 | 252 | 1350 | 1,3-GDO 42 mg (1 mole) | 0.5 |
| Example 3-2 | S-021 | 252 | 1350 | 1,3-GDO 210 mg (5 moles) | 0.5 |

In order to confirm the effect of suppressing the initial burst in microspheres including 0.1 moles of 1,3-GDO (Example 3-1), microspheres including 1 mole of 1,3-GDO (Example 2-1), and microspheres including 5 moles of 1,3-GDO (Example 3-2) according to Table 5, the content and release rate of semaglutide (API) were confirmed under the conditions of Test Methods 1 and 2, the surfaces of the microspheres were observed using SEM photographs under the conditions of Test Methods 3 and 4, and the average particle size (D[4,3]) of each of the microspheres was measured.

In addition, the physicochemical properties (bulk density (BD), zeta potential) of each of the microspheres were measured using Test Methods 5 and 6.

The results are shown in the following Table 6 and FIG 3.

FIGS. 3A and 3B are SEM photographs of microspheres prepared according to an exemplary embodiment of the present invention. Specifically, FIG 3A shows microspheres according to Example 3-1, and FIG 3B shows microspheres according to Example 3-2.

**[Table 6]**

| | **Batch No.** | **Analysis results** | | | | | |
|---|---|---|---|---|---|---|---|
| | | **Content (%)** | **Release (1h) (%)** | **D[4,3] (µm)** | **SEM properties** | **Zeta value (mV)** | **BD (g/ml)** |
| Example 3-1 | S-020 | 97.95 | 6.17 | 29.9 | No pores (coating) | -17.5 | 0.314 |
| Example 2-1 | S-018 | 107.93 | 6.91 | 45.11 | No pores (coating) | -11.7 | 0.229 |
| Example 3-2 | S-021 | 96.78 | 8.47 | 33.5 | No pores (coating) | -27.8 | 0.26 |

As shown in Table 6 and FIGS. 2C, 3A and 3B, it can be seen that when GDO as a lipid component was used in the microspheres in a range of 0.1 moles (Example 3-1), 1 mole (Example 2-1) and 5 moles (Example 3-2), almost no pores were observed on the surface because the pores of the microspheres were coated, and it can be confirmed that the properties were also good while having the effect of suppressing the initial burst of API.

### [Test Example 4] Confirmation of effect of suppressing release control depending on content of 1,3-GDO and cholesterol

In order to confirm whether the effect of suppressing the release of the drug in microspheres including semaglutide as a main component from the microspheres is caused by the content of the lipid component, the release of API from the microspheres and the properties of the microspheres depending on the content of 1,3-GDO and cholesterol were confirmed.

Microspheres were prepared with the ingredients and contents shown in the following Table 7. The method for preparing the microspheres is the same as that for preparing the microspheres in Preparation Example 1.

**[Table 7]**

| | **Batch No.** | **Composition** | | | |
|---|---|---|---|---|---|
| | | **W1 Phase (mg)** | **O Phase (mg)** | | **W2 Phase (g)** |
| | | API | Polymer | Lipid | PVA |
| Example 4-1 | S-022 | 252 | 1350 | 1,3-GDO 4.2 mg (0.1 moles) + Cholesterol 2.6 mg (0.1 moles) | 0.5 |
| Example 4-2 | S-023 | 252 | 1350 | 1,3-GDO 210 mg (5 moles) + Cholesterol 2.6 mg (0.1 moles) | 0.5 |
| Example 2-2 | S-019 | 252 | 1350 | 1,3-GDO 42 mg (1 moles) + Cholesterol 26 mg (1 moles) | 0.5 |
| Comparative Example 4-1 | S-024 | 252 | 1350 | 1,3-GDO 4.2 mg (0.1 moles) + Cholesterol 130 mg (5 moles) | 0.5 |
| Example 4-3 | S-025 | 252 | 1350 | 1,3-GDO 210 mg (5 moles) + Cholesterol 130 mg (5 moles) | 0.5 |

In order to confirm the effect of suppressing the initial burst in microspheres including 0.1 moles of 1,3-GDO and 0.1 moles of cholesterol (Example 4-1), microspheres including 5 moles of 1,3-GDO and 0.1 moles of cholesterol (Example 4-2), microspheres including 1 mole of 1,3-GDO and 1 mole of cholesterol (Example 2-2), microspheres including 0.1 moles of 1,3-GDO and 5 moles of cholesterol (Comparative Example 4-1), and microspheres including 5 moles of 1,3-GDO and 5 moles of cholesterol (Example 4-3), the content and release rate of semaglutide (API) were confirmed under the conditions of Test Methods 1 and 2, the surfaces of the microspheres was observed using SEM photographs under the conditions of Test Methods 3 and 4, and the average particle size (D[4,3]) of each of the microspheres was measured.

In addition, the physicochemical properties (bulk density (BD), Zeta potential) of each of the microspheres were measured using Test Methods 5 and 6.

The results are shown in the following Table 8 and FIG 4. FIGS. 4A to 4D are SEM photographs of microspheres prepared according to an exemplary embodiment of the present invention. Specifically, FIG 4A shows microspheres according to Example 4-1, FIG 4B shows microspheres according to Example 4-2, FIG 4C shows microspheres according to Comparative Example 4-1, and FIG 4D shows microspheres according to Example 4-3.

**[Table 8]**

| | **Batch No.** | **Analysis results** | | | | | |
|---|---|---|---|---|---|---|---|
| | | **Content (%)** | **Release (1h) (%)** | **D[4,3] (µm)** | **SEM properties** | **Zeta value (mV)** | **BD (g/ml)** |
| Example 4-1 | S-022 | 102.07 | 0.1 | 49.4 | No pores (coating) | -15.8 | 0.36 |
| Example 4-2 | S-023 | 100.12 | 6.34 | 30.4 | No pores (coating) | -30.1 | 0.251 |
| Example 2-2 | S-019 | 109.42 | 6.56 | 56.68 | No pores (coating) | -13.8 | 0.279 |
| Compara tive Example 4-1 | S-024 | 106.52 | 13.28 | 54.9 | Poor properties | -11.8 | 0.219 |
| Example 4-3 | S-025 | 96.94 | 5.67 | 32.8 | Excellent properties | -23.9 | 0.246 |

As shown in Table 8 and FIGS. 2D and 4A to 4D, it can be confirmed that the microspheres using 0.1 moles of GDO and 5 moles of cholesterol as lipid components (Comparative Example 4-1) have poor properties and also show a large initial burst of API. That is, 1,3-GDO and cholesterol need to be used as the lipid components at a molar ratio of less than 1:50.

However, when cholesterol and 1,3-GDO are used as the lipid components at a molar ratio of 1:0.002 to less than 1:50 as in Examples 4-1, 4-2, 2-2, and 4-3, almost no pores are confirmed on the surface because the pores of the microspheres are covered. Through this, it can be confirmed that properties are also good while having the effect of suppressing the initial burst of API.

### [Test Example 5] Confirmation of initial burst control and sustained release effect in vivo

An experiment was carried out to confirm the drug release behavior of microspheres *in vivo.*

After microspheres (0.4 mg/kg semaglutide) prepared according to Example 2-2 and Comparative Example 2-1 in Table 3 were subcutaneously administered to the backs of four 8-week-old SD rats (male) weighing an average of 300 g, blood was collected on days 0, 0.04, 0.13, 0.25, 0.5, 1, 2, 4, 7, 14, 24, and 28.

Thereafter, the concentration of semaglutide in the blood at each time point was measured for the plasma samples of SD rats using enzyme-linked immunosorbent assay (ELISA), and as a kit used, GLP-1 (active) ELISA (IBL, Germany) was used.

The change in semaglutide concentration over time is shown in FIG 5. The results shown in FIG 5 describe the average values for the four rats used in the experiment.

As shown in FIG 5, the microspheres (Example 2-2) coated with 1 mole each of GDO and cholesterol relative to the main component (API) showed a 2.6-fold decrease in maximum blood concentration (Cmax) of the physiologically active substance and a 4-fold delay in maximum blood time (Tmmx) compared to the microspheres of Comparative Example 2-1, which are in the general form of fine particles. That is, it can be confirmed that the microspheres according to the present invention exhibited an excellent sustained release effect from 21 days to 28 days.

### [Industrial Applicability]

The microspheres of the depot composition of the present invention can control an active ingredient included in microspheres from being released in an excessive amount at the beginning of the injection of the depot preparation by including a lipid component, and have excellent suspensibility, so that even when the depot composition of the present invention is administered as an injection by a user, the effect of the active ingredient can be obtained uniformly and continuously.

## Claims

1. A depot composition comprising microspheres comprising an oil layer (O layer) comprising a lipid component and a biodegradable polymer.

2. The depot composition of claim 1, wherein the lipid component is glycerol dioleate (GDO) or tripalmitin, or a mixture of cholesterol and a second lipid component.

3. The depot composition of claim 2, wherein the second lipid component is one or more selected from the group consisting of GDO, 1,2-dioleoyl-3-trimethylammonium-propane (DOTAP) and tripalmitin.

4. The depot composition of claim 1, wherein the microspheres are prepared by comprising the lipid component in an amount of 20 parts by weight or less with respect to 100 parts by weight of the biodegradable polymer.

5. The depot composition of claim 2, wherein cholesterol is comprised in an amount of more than 0.001 moles and less than 50 moles with respect to 1 mole of the second lipid component.

6. The depot composition of claim 1, wherein the biodegradable polymer is a polymer comprising lactide and glycolide as monomers.

7. The depot composition of claim 1, wherein the biodegradable polymer has an inherent viscosity (25°C) of 0.35 dL/g to 0.65 dL/g.

8. A method for preparing a depot composition, the method comprising: preparing an oil phase (O) solution comprising a lipid component and a biodegradable polymer, or preparing a W1/O emulsion by mixing a first water phase (W1) solution comprising a water-soluble solvent with the oil phase (O) solution; and
preparing an O/W2 emulsion or a W1/O/W2 emulsion by inputting the oil phase solution or W1/O emulsion into a second water phase 2 (W2) solution.

9. The method of claim 8, wherein the lipid component is comprised in an amount of 20 parts by weight or less with respect to 100 parts by weight of the biodegradable polymer.

10. The method of claim 8, wherein the first water phase and/or oil phase further comprise an active ingredient, and
the lipid component is comprised in an amount of 0.01 to 10 moles with respect to 1 mole of the active ingredient in the oil phase solution.

11. The method of claim 8, further comprising recovering microspheres by drying and/or filtering the prepared O/W2 emulsion or W1/O/W2 emulsion and then centrifuging the emulsion.
